# EUROPEAN PATENT APPLICATION

(11) **EP 4 681 597 A1**
(43) Date of publication of application: **21.01.2026**
(21) Application number: 24306207.2
(22) Date of filing: 18.07.2024
(51) Int. Cl.: A61B 3/00, A61B 3/10, A61B 3/107

(54) **SYSTEM FOR DETERMINING A CHARACTERIZATION OF AT LEAST ONE PARAMETER OF AN EYE MODEL**

(71) Applicant: Essilor International, 94220 Charenton-Le-Pont (FR)
(72) Inventor: MESTRE, Michael, 91120 PALAISEAU (FR); SVERDLIN, Julia, 75005 PARIS (FR)
(74) Representative: Plasseraud IP

(57) **Abstract**

A system (100) for determining a characterization of at least one parameter of an eye model for a patient's eye (101), comprises an optical coherence tomography device, an aberrometer and a double-pass channel (10) which is common to the optical coherence tomography device and the aberrometer. An optical direction that is common to an illumination beam (B0) injected into the patient's eye and to a return beam (BR) recovered from the patient's eye can be varied. The characterization is determined from measured values of an optical path length and measured features of a wavefront relating to at least two angular positions of the common optical direction. Using at least two angular positions for the common optical direction increases accuracy and reliability of the determined characterization.

## Description

The present disclosure relates to a system and a method for determining a characterization of at least one parameter of an eye model.

### -- BACKGROUND --

A human eye is a complex optical system which involves numerous optical and geometrical parameters, including the axial length, retinal curvature, corneal and lens curvatures, refractive index of the light-transmitting media of the eye, accommodation dynamics and response curves during an eye exam, evolution of the retinal shape over months, gradient index of the crystalline lens, etc. At least some of these parameters can be integrated into an eye model. For example, Navarro's eye model is well-known but other eye models exist which are more complex.

Having characterizations of the parameters that relate to each patient is useful for many applications, including providing optimized ametropia correction and/or myopia control eyewear to the patient. Other applications may relate to eye surgery, in particular for allowing a surgeon to prepare an operation of the patient's eye.

Systems such as aberrometers and optical coherence tomography systems, also referred to as low-coherence reflectometers, are used for making measurements on an eye, so as to determine characterizations for the parameters of an eye model that is implemented for describing the eye. But it is generally difficult to converge towards correct characterizations, in particular when a large number of parameters is involved in the eye model and/or because of ambiguities such as higher curvature of a refracting surface versus shorter optical path. Because of these reasons, it has been proposed to combine several optical measurement types within one and same ocular measurement system to better determine characterizations for the parameters of an eye model. However, existing systems are still not satisfactory and there remains a need to characterize eye model parameters in a more precise and reliable way.

US 2023/0064504 A1 discloses a system for combined intraoperative aberrometry and optical coherence tomography which enables the integration of both optical coherence tomography and wavefront aberrometry within a single device such as a surgical microscope, a clinical biometry device, and/or other system. This combined system uses a single, shared light source, beam delivery system, and control unit, with two separate detection channels, to generate both optical coherence tomography images and aberration measurements. The aberration measurements are limited to on-axis aberration measurements, i.e. limited to measuring aberrations when the illumination spot is focused on a central point of the eye retina. In addition, the optical coherence tomography subsystem acquires images when the aberrometer is inactive, and the aberrometer performs sensing when the optical coherence tomography subsystem is inactive.

The present specification addresses the need of characterizing eye model parameters for a patient, in a more precise and reliable way.

### -- SUMMARY --

For meeting this object or others, a first aspect of the present disclosure proposes a system for determining a characterization of at least one parameter of an eye model for a patient's eye, which system comprises:
- an optical coherence tomography device,
- an aberrometer, and
- a double-pass channel, which is common to the optical coherence tomography device and the aberrometer.

According to a first feature of the system, the double-pass channel is arranged for injecting an illumination beam into the patient's eye and recovering a return beam from the patient's eye both parallel to a common optical direction, during a system use.

The optical coherence tomography device is configured for providing measured values of an optical path length, and the aberrometer is configured for providing measured features of a wavefront.

According to a second feature of the system, the system is configured for determining the characterization of the at least one parameter from the measured values of the optical path length and the measured features of the wavefront relating to at least two angular positions of the common optical direction.

Each characterization that is obtained using the system for any parameter of the eye model is thus personalized for the patient. In various embodiments, the characterization of each parameter of the eye model as determined by the system for the patient's eye may be a value or a statistical estimator of this parameter.

The eye model may be an optical eye model. The system may be an optical system.

The system allows measuring both the optical path length and the wavefront for a plurality of angular positions of the illumination beam and return beam. The plurality of angular positions for the common optical direction corresponds to intersecting the retina of the patient's eye successively at several points. At least one of these angular positions is off-axis, i.e. the common optical direction intersects the retina of the patient's eye at a non-zero distance from a central point of the retina. Thanks to such off-axis angular positions of the common optical direction, the measurement set that is used for each characterization of the at least one parameter is thus enriched with additional measurement information. This enriched measurement information allows more precise, accurate and reliable characterization of each parameter. In particular, the measurement types provided by the system, i.e. off-axis optical coherence tomography combined with off-axis aberrometry, strongly reduces ambiguities that might exist otherwise between several values for one and same eye model parameter.

The system is thus designed for performing both an optical coherence tomography measurement, i.e. providing a measured optical path length value, and an aberrometry measurement, i.e. providing measured wavefront features, for each of a plurality of angular positions of the common optical direction. Most preferably, these measured optical path length values and measured wavefront features may be obtained from captures that are simultaneous for each angular position of the common optical direction. Measurement time is thus reduced, and this also increases the accuracy of each parameter characterization that is obtained.

Preferably, the system may further comprise:
- an ametropia-compensating device arranged on an optical path segment that is common to a part of the illumination beam and to a part of the return beam which propagates to an interferometer and detector arrangement of the optical coherence tomography device, and such that the illumination beam is focused onto the retina of the patient's eye during the system use.

In particular, the ametropia-compensating device increases accuracy of the optical coherence tomography measurements. Advantageously, the ametropia-compensating device may be located between the interferometer and detector arrangement and the double-pass channel. Possibly, the system may further comprise a beam divider arranged for directing a further part of the return beam to the interferometer and detector arrangement of the optical coherence tomography device, and directing another further part of the return beam to a wavefront analyser of the aberrometer. Such beam divider may be located on an optical path segment between the ametropia-compensating device and the double-pass channel. Such location avoids stray light produced by reflections on optical components of the double-pass channel.

In preferred embodiments of the system, the double-pass channel may comprise:
- a scanning device at an entrance pupil of the double-pass channel, suitable for varying between the at least two angular positions of the common optical direction; and
- an afocal optical device located between the entrance pupil and a sample pupil of the double-pass channel dedicated to being superimposed on a pupil of the patient's eye during the system use, so as to transmit both the illumination beam and the return beam between the entrance pupil and the sample pupil.

Generally, the system may advantageously further meet at least one of the following optional features:
- the optical coherence tomography device may comprise an interferometer of a Michelson type;
- the aberrometer may comprise a wavefront analyser of a Shack-Hartmann type;
- the system may comprise an illumination channel common to both the optical coherence tomography device and the aberrometer, this illumination channel being suitable for producing the illumination beam. Additionally, at least part of this illumination channel may comprise an optical fiber-based assembly; and
- the system may be configured for controlling a variation of the common optical direction according to a continuous motion that comprises successive superpositions of the common optical direction with a central optical axis of the double-pass channel, and the measured values of the optical path length and the measured features of the wavefront are measured at least one time between two successive superpositions of the common optical direction with the central optical axis.

In first embodiments of the system, the system may be configured for:
repeatedly implementing the eye model according to tested characterizations of the at least one parameter, for calculating test results that comprise calculated values of the optical path length and calculated features of the wavefront for the at least two angular positions of the common optical direction; and
determining the characterization of the at least one parameter of the eye model from a best-match search carried out between:
   - a target that comprises the measured values of the optical path length and the measured features of the wavefront for the at least two angular positions of the common optical direction, and
   - the test results.

For such first embodiments, the system may be further configured for carrying out the best-match search by computing values of a cost function that quantifies a discrepancy between the target and the test results.

In second embodiments of the system, the system may be configured for implementing a relationship providing the characterization of the at least one parameter of the eye model from the measured values of the optical path length and the measured features of the wavefront relating to the at least two angular positions of the common optical direction.

According to a possible improvement, the system may be configured for determining the characterization of the at least one parameter, from statistical data that relate to characterizations possible for the at least one parameter of the eye model, clinical data of the patient and/or additional measurement data collected for the patient, in addition to the measured values of the optical path length and the measured features of the wavefront relating to the at least two angular positions of the common optical direction. Possibly, the statistical data may include probability values and/or covariance values relating to the at least one parameter of the eye model.

According to another possible improvement, the system may be configured for determining the characterization of the at least one parameter separately for several eye models from one and the same measurement sequence executed for the patient's eye, and outputting respective values of a residual discrepancy for the eye models, each of the values of the residual discrepancy quantifying for the corresponding eye model a discrepancy between:
- modelled values of the optical path length and modelled features of the wavefront for the at least two angular positions of the common optical direction as resulting from inputting into the eye model the characterization of the at least one parameter obtained for this eye model, and
- the measured values of the optical path length and the measured features of the wavefront for the at least two angular positions of the common optical direction.

Then, the system is further configured for selecting one of the eye models that has the value of the residual discrepancy corresponding to a least discrepancy.

A second aspect of the present disclosure proposes a method for determining a characterization of at least one parameter of an eye model for a patient's eye, which method comprises:
/i/ providing a system according to the first aspect of the present disclosure, including its alternative embodiments and optional improvements;
/ii/ positioning the patient's eye with respect to the system and collecting the respective measured values of the optical path length and the respective measured features of the wavefront for the at least two angular positions of the common optical direction; and
/iii/ from input data that comprise the measured values of the optical path length and the measured features of the wavefront for the at least two angular positions of the common optical direction, determining the characterization of the at least one parameter of the eye model that relate to the patient's eye.

Preferably, a number of the at least two angular positions of the common optical direction may be comprised between ten and one hundred.

When the system is configured for implementing a relationship that provides the characterization of the at least one parameter of the eye model from the measurement results, this relationship may have been obtained by a neural network previously trained with data of at least one of the following types: simulated data and experimental data.

In possible implementations of the method, the at least one parameter may describe an accommodation state of the patient's eye, a shape of a retina of the patient's eye, or a refractive index gradient of a crystalline lens of the patient's eye.

These and other features will be now described with reference to the appended figures, which relate to preferred but not-limiting embodiments.

### -- BRIEF DESCRIPTION OF THE DRAWINGS --

Figure 1 displays a general arrangement of systems for determining a characterization of parameters of an eye model for a patient's eye; and
Figure 2 displays a detailed optical arrangement of a system according to Figure 1.

For clarity's sake, element sizes which appear in these figures do not correspond to actual dimensions or dimension ratios, and some of the elements are only represented symbolically. Also, same reference numbers which are indicated in both figures denote identical elements of elements with identical function.

### -- DETAILED DESCRIPTION --

The references now listed have the following meanings:
- 101: eye of a patient, for which eye model parameter characterizations are to be determined
- R, OP: retina and pupil of the eye 101, respectively
- 100: system for determining the characterizations of the eye model parameters for the eye 101
- 10: double-pass channel
- 11: scanning mirror
- 12: two-axis actuator, for varying an orientation of the scanning mirror 11
- 13: sensor, for detecting the instant orientation of the scanning mirror 11
- 14: afocal optical device
- 20: illumination and OCT-detection channel, OCT standing for optical coherence tomography
- 21: light source, forming the illumination channel mentioned in the general part of the present description
- 22: interferometer
- 23: spectrometer or a fast photodetector
- 30: ametropia-compensating device
- 40: wavefront analyser
- 50: data processing unit
- 60: beam divider

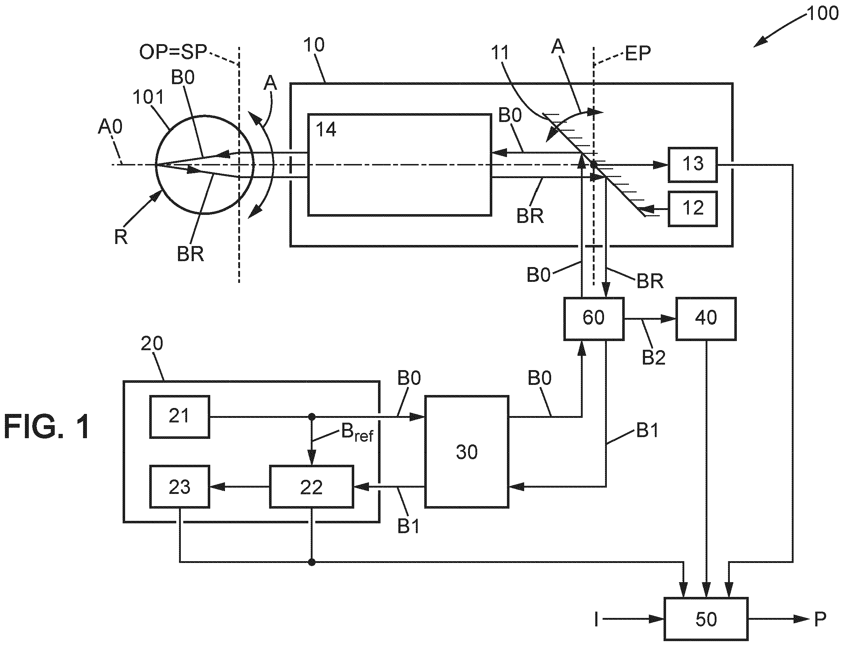

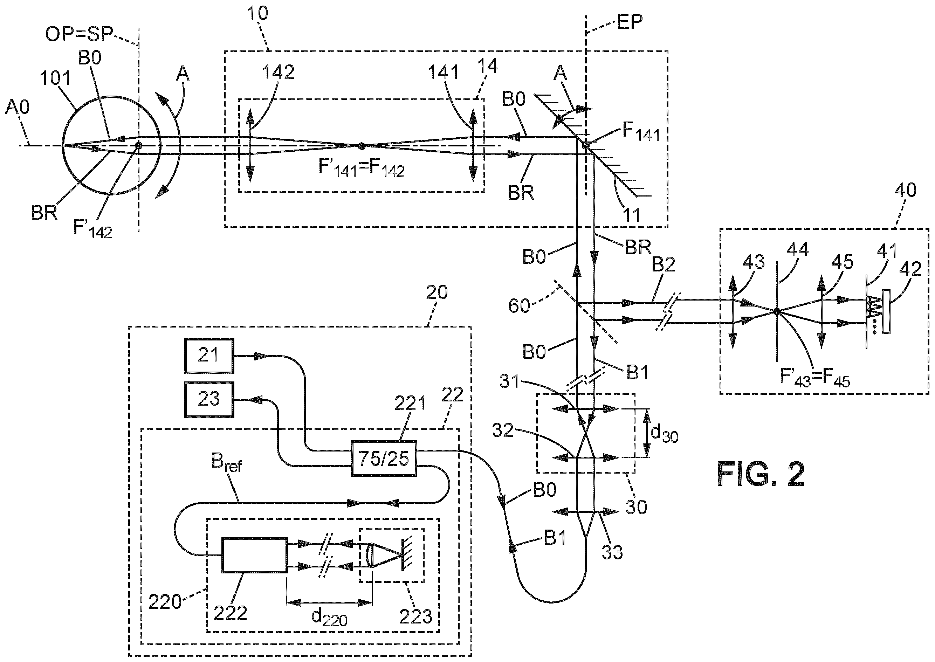

In Figures 1 and 2, the illumination and OCT-detection channel 20 comprises the light source 21, the interferometer 22 and the spectrometer 23. The light source 21 has low time-coherence, suitable for operation of the optical coherence tomography device, also known as low-coherence reflectometer. The light source 21 transmits an illumination light beam B0 to the double-pass channel 10. Simultaneously, the illumination and OCT-detection channel 20 receives a first part B1 of a return light beam BR, and transmits it to the spectrometer 23 through the interferometer 22. The interferometer 22 and the spectrometer 23 together form the interferometer and detector arrangement of the low-coherence tomography device introduced in the general part of the present description.

The illumination beam B0 is backscattered by the retina R of the patient's eye 101 during a use of the system 100, thus producing the return beam BR. The return beam BR propagates back through the double-pass channel 10 and exits therefrom in superposition with the illumination beam B0 but propagating in opposite direction.

The double-pass channel 10 comprises the scanning mirror 11 associated with the actuator 12 and the sensor 13 for forming a two-axis scanning device, and further comprises the afocal optical device 14. The scanning mirror 11 may be replaced by two one-axis rotating mirrors which may be conjugated through an intermediate imaging optics. EP and SP denote respectively an entrance pupil and an exit pupil of the afocal optical device 14. In the present embodiment, the entrance pupil EP of the afocal optical device 14 forms the entrance pupil of the double-pass channel 10. The exit pupil SP is to be superimposed with the pupil OP of the eye 101 during the use of the system 100 with the patient's eye 101, and it is referred to as sample pupil from now. The diameter of the sample pupil SP may be at most that of the pupil OP of the eye 101 of the patient. The scanning mirror 11 is located at the entrance pupil EP. Thus, varying the orientation of the scanning mirror 11 by controlling the actuator 12 causes an instant angular position of an optical direction that is common to both the illumination beam B0 and the return beam BR to vary through the sample pupil SP. The instant angular position of the common optical direction is identified by the sensor 13, and communicated to the data processing unit 50.

The data processing unit 50 may comprise a memory and a processor. The data processing unit 50 may be a low resource data processing unit.

Examples of processors include microprocessors, microcontrollers, graphics processing units (GPUs), central processing units (CPUs), application processors, digital signal processors (DSPs), reduced instruction set computing (RISC) processors, systems on a chip (SoC), baseband processors, field-programmable gate arrays (FPGAs), programmable logic devices (PLDs), state machines, gated logic, discrete hardware circuits, and other suitable hardware configured to perform the various functionalities described throughout this disclosure.

The memory is a computer-readable media. By way of example, and not limitedly, such computer-readable media may include a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer-executable code in the form of instructions or data structures that may be accessed by the processor of the data processing unit 50.

The beam divider 60 splits the return beam BR into first and second parts thereof. The first part, which is denoted B1, propagates back to the interferometer and detector arrangement, and the second part, denoted B2, is directed to the wavefront analyser 40. The light source 21, the double-pass channel 20 and the wavefront analyser 40 form an aberrometer.

The ametropia-compensating device 30 is arranged on the optical path segment of both the illumination beam B0 and the first part B1 of the return beam BR, preferably between the illumination and OCT-detection channel 20 and the beam divider 60. This ametropia-compensating device 30 ensures that the illumination beam B0 is focused on the retina R of the eye 101, so that a single optical path length value is defined and can be outputted by the optical coherence tomography device formed by the light source 21, the double-pass channel 10, the interferometer 22 and the spectrometer 23.

Within the illumination and OCT-detection channel 20, a part B_{ref} of the light beam produced by the light source 21 is directed to the interferometer 22, in addition to the illumination beam B0 transmitted toward the eye 101 through the above-mentioned optical components. The interferometer 22 makes the light beam part B_{ref} propagate along a reference arm having controlled length, and mixes this light beam part B_{ref} returning from the reference arm with the first part B1 of the return beam BR. The spectrometer 23 allows comparison between the respective optical path lengths of the light beam part B_{ref} and of the first part B1 of the return beam BR in a well-known manner for optical coherence tomography, also called low-coherence reflectometry. So-constituted optical coherence tomography device provides the instant optical path length value that relates to the illumination beam B0 followed by the first part B1 of the return beam BR. When varying the orientation of the scanning mirror 11, each instant optical path length value is to be associated with the angular position of the common optical direction of both the illumination beam B0 and the return beam BR at the sample pupil SP as effective at the same time. Such multiple angular positions of the common optical direction, including off-axis positions, provide enriched information about the model shape of the retina R. A-labelled arrows in the figures show corresponding variations of the orientation of the scanning mirror 11 and of the common optical direction of the illumination beam B0 and return beam BR at the sample pupil SP.

In a known manner, the illumination and OCT-detection channel 20 may have other compositions while still having an optical coherence tomography operation, for example using a fast-sweeping LED, a swept-source LASER or a supercontinuum LASER as the light source 21 and a photodiode as the detector instead of the spectrometer 23. More specifically, when the light source 21 can perform a wavelength scan, such as a swept source, the spectrometer 23 may be replaced by a fast photodetector, such as a fast photodiode, which is operated in a manner synchronized with the wavelength scan carried out by the swept source.

The aberrometer simultaneously outputs, also for each common optical direction of the illumination beam B0 and return beam BR at the sample pupil SP, measured features of the wavefront shape of the return beam BR, preferably as existing at the sample pupil SP superimposed with the eye pupil OP. These measured wavefront features may be of a type which depends on the wavefront analyser that is actually used, or may be geometrical parameters that characterize the wavefront shape. For example, such geometrical parameters may be local curvatures, local tilt and/or piston values, or local sagittal heights of the wavefront.

During the above operation of the system 100 with the eye 101, the eye 101 is maintained fixed in orientation relative to the double-pass channel 10. Put another way, the eye 101 remains fixed with its eye axis superimposed to a central optical axis A0 of the double-pass channel 10. Suitable eye orientation fixing device is well-known in ophthalmics and not represented in the figures. For example, it may use an additional visible light source acting as a visual point target, and a control camera for checking the instant eye orientation and discarding measurement results not corresponding to straight-ahead eye orientation.

According to a possible improvement of the system 100 which reduces unwanted rotations of the eye 101, the movements of the scanning mirror 11 may be controlled so that the common optical direction of the illumination beam B0 and return beam BR runs along a multiple-loop path at the sample pupil SP, and at least one measurement time in each loop is used for simultaneous operations of the aberrometer and the optical coherence tomography device. Maximal angle deviation of the common optical direction from the central optical axis A0 of the double-pass channel 10 may be of about 15° (degree), for example, and the common optical direction comes back to the central optical axis A0 of the double-pass channel 10 each 0.3 second maximum, advantageously each 50ms (millisecond) maximum. Possibly, the movement of the scanning mirror 11 may be temporarily stopped for executing simultaneously the measurement operations of both the optical coherence tomography device and the aberrometer, and the movement is resumed straight after.

The data processing unit 50 receives measurement data from the spectrometer 23 and from the wavefront analyser 40 for each position of the scanning mirror 11. Possibly, it is further supplied with additional data I such as statistical data, clinical data relating to the patient and additional measurement data obtained from other measurement systems, as non-limiting examples for such additional data I. Some of these data may be supplied by the patient himself. For example, the statistical data may relate to a patient group in the form of probability values for possible eye model parameter values and covariance values connecting pairs of possible eye model parameter values. The measured values of the optical path length and the measured wavefront features collected for the plurality of implemented angular positions of the common optical direction constitute with the additional data I if any, the input data of the data processing unit 50 as mentioned in the general part of this description. They are used for determining the characterization of each parameter of the eye model.

Each characterization that is obtained using the system for any parameter of the eye model is thus personalized for the patient. In various embodiments, the characterization of each parameter of the eye model as determined by the system for the patient's eye may be a value or a statistical estimator of this parameter. The values may be personalized values P.

The data processing unit 50 thus outputs respective values P for the eye model parameters, that are personalized for the patient. Possibly in a known manner, the data processing unit 50 may output also a personalized statistical estimator for each parameter of the eye model, instead of only the personalized value P for this parameter. Statistical estimators and their implementation are well-known by the skilled person.

The data processing unit 50 may proceed in several ways for determining the personalized values P of the eye model parameters. According to a first operation possible for the data processing unit 50, it may execute the following steps:
/1/ supplying a set of initial parameter values based on the additional data I or any other initialization process;
/2/ for each angular position of the common optical direction produced with the scanning mirror 11, inputting the initial parameter values into the eye model and calculating the optical path length value and the wavefront shape at the sample pupil SP, using an optical system model for the system 100;
/3/ calculating discrepancy components that quantify each a discrepancy between the calculated and measured optical path length values and also between the calculated and measured wavefront shapes, a separate discrepancy component respectively for each of several angular positions of the common optical direction of the illumination beam B0 and return beam BR;
/4/ calculating a cost function, also known as merit function, as a sum of the discrepancy components corresponding to the plurality of angular positions of the common optical direction of the illumination beam B0 and return beam BR, and possibly further adding other terms originating from the additional data I;
/5/ repeating steps /2/ to /4/ with tested parameter values different from the initial ones, selecting one of the sets of the initial or tested parameter values that leads to minimal result for the cost function, and replacing the initial parameter values with the selected parameter values; and
/6/ repeating step /5/ iteratively until a convergence criterium is met or a threshold value is reached for the cost function result.

Such optimization process of best-match search type is well-known in the art. It suits for determining simultaneously up to twenty eye model parameters or more, including Zernike parameters corresponding to polynomial orders less than or equal to five, for example less than or equal to four. In particular, the so-determined eye model parameters can provide numeral topography descriptions of the retina R and also of the anterior part of the eye 101, including information relating to the cornea and crystalline lens surface shapes. The wavefront shapes and optical path lengths calculated in the above steps /2/ and /4/ using the eye model with the initial or currently tested values of the eye model parameters have been called test results in the general part of the present description.

The minima of the cost function can be explored by implementing various processes for selecting the new tested parameter values. In particular, selection processes such as simulated annealing or based on a neural network can be used.

According to a second operation also possible for the data processing unit 50, it may implement a relationship that connects the measurement results, namely the measured optical path length values outputted by the optical coherence tomography device and the corresponding measured wavefront features outputted by the aberrometer for several angular positions of the scanning mirror 11, to values of the parameters of the eye model. The relationship may have been obtained beforehand by a suitable computing system, preferably of neural network type. In particular, the relationship may have been obtained from a training process from labelled data, collected from simulations or experimentally or from any combination of simulation-obtained and experimentally obtained labelled data.

According to an optional improvement of the data processing unit 50, it may be configured for implementing separately several eye models, and thereafter selecting that one among these eye models which provides a best match with the measurement results. Such best match may be determined by using a discrepancy quantifying function that quantifies separately for each eye model which has been implemented, a residual discrepancy that exists between the measurement results and modelled values calculated by inputting the personalized values P of the parameter into the corresponding eye model, for the optical path length and the corresponding wavefront features for the plurality of angular positions of the scanning mirror 11. Possibly, a cost function may be used as the discrepancy quantifying function for such eye model selection, which is similar to that used in the above-described first possible operation of the data processing unit 50.

The system 100 may be used for several applications.

First applications may relate to measuring accommodation dynamics and response curves during an exam of the patient's eye 101. To this end, one or several parameters of the eye model describe(s) a current accommodation state of the crystalline lens of the eye 101, such as in Navarro eye model. For example, one of these parameters is a curvature of the crystalline lens. A single operation of the system 100 with the patient's eye 101 thus provides a characterization of the current accommodation state. Multiple operations repeated successively with a controlled variation such as a variable axial position of a fixation target provide a trajectory of accommodation states in a manner of an accommodative stimulus-response curve. Accommodation dynamics with associated fluctuations around equilibrium response can be characterized in this way just-in-time or in real-time.

Second applications may relate to the evolution of the retinal shape over months, in particular for assessing efficiency of myopia management lenses in children. To this end, one or several parameters of the eye model describe(s) a deviation of the retina R from a spherical shape, called oblateness. Indeed, oblateness variation indicates myopia progression, and can be characterized using the system 100 every few months. Possibly, characterizations of the axial length of the eye 101 may supplement the oblateness characterizations. The Atchison eye model can be used for such second applications.

Third applications may relate to measuring the refractive index gradient of the crystalline lens of the eye 101 in order to identify pathologies. Indeed the fine index gradient structure of the crystalline lens has a major role in the spherical aberration on the retina R, and it is prone to a number of pathologies and defects that can greatly impact vision. The system 100 allows routine exams for screening these pathologies.

Figure 2 illustrates possible embodiments for the optical components of the system 100.

The afocal optical device 14 may comprise two optical objectives 141 and 142 which may be identical to each other, and which are represented as converging lenses for simplification. Then, the central optical axis A0 is the rotation-symmetry axis which is common to both objectives 141 and 142. The object focal point F₁₄₁ of the objective 141 is superimposed on the scan mirror 11 and the image focal point F'₁₄₁ of the same objective 141 is superimposed on the object focal point F₁₄₂ of the objective 142. The location of the image focal point F'₁₄₂ of the objective 142 then defines the location of the sample pupil SP of the afocal optical device 14. The objectives 141 and 142 may have optimized design for reducing optical aberrations of the afocal optical device 14. This reduction of the optical aberrations may be on-axis and throughout the entire field.

The ametropia-compensating device 30 may comprise two converging lenses 31 and 32 which have respective focal points superimposed on each other between both lenses if the eye 101 has no ametropia. A separating distance d₃₀ between both lenses 31 and 32 is to be adjusted for compensating the ametropia of the eye 101 if any. Other well-known optical arrangements may be used alternatively for the ametropia-compensating device 30.

The beam divider 60 may comprise a semi-reflecting plate which is effective for the emission range of the light source 21, but other beam dividers may be used alternatively. The beam divider 20 may comprise a pellicle beam splitter.

The light source 21 may be a superluminescent LED, commonly referred to as SLED, for example with central emission wavelength of about 830 nm (nanometer) and emission bandwidth of about 20 nm. It is connected to an optical input port of the interferometer 22.

The spectrometer 23 may be of any type known in the art suitable for the spectral range of the light source 21, preferably based on a spectrally spreading element such as a prism or a grating. It is connected to an optical output port of the interferometer 22.

The lens 33 has a condenser function for optically connecting the ametropia-compensating device 30 to an optical input-output port of the interferometer 22. Thus, the ametropia-compensating device 30, the double-pass channel 10 and the eye 101 form a sample arm of the interferometer 22.

The illumination and OCT-detection channel 20 is preferably based on optical fiber technology, for example using single mode optical fibers. Then, ports of a first side of a 2 x 2 fiber-based optical coupler 221 form the above-mentioned optical input and output ports of the interferometer 22. Ports of a second side of the 2 x 2 fiber-based optical coupler 221 are separate optical input-output ports of the interferometer 22. One of them is optically connected to the condenser lens 33, and the other one to a reference arm 220 of the interferometer 22. All optical connections to the 2 x 2 optical coupler 221 are implemented with optical fibers, preferably of multimode type but not limitedly. The 2 x 2 optical coupler 221 may be selected so as to transmit about 75% of the light power produced by the light source 21 to the reference arm 220, and the remaining 25% to the sample arm as the illumination beam B0. B_{ref} denotes the beam part that is transmitted by the optical coupler 221 from the light source 21 to the reference arm 220, and is called reference beam. According to the well-known operation of the optical coherence tomography device, the reference arm 220 may be designed for producing a variable reference optical path length. As an example, the reference arm 220 may comprise a collimator 222 and a retroreflecting assembly 223 as illustrated, with variable separating distance d₂₂₀ between the collimator 222 and retroreflecting assembly 223. Possibly, the retroreflecting assembly 223 may comprise a condenser lens and a mirror fixedly located at the focus point of the condenser lens, but other retroreflecting assemblies are possible alternatively such as a cube-corner three-mirror assembly. The distance d₂₂₀ that produces maximum contrast value for an interference state captured by the spectrometer 23 constitutes a measurement of the optical path length of the sample arm. The interferometer 22 in the optical coherence tomography device just described is of Michelson type, but other interferometer types may be used alternatively.

The aberrometer may be based on a Shack-Hartmann wavefront analyser 40, but other wavefront analysers may be used alternatively. The Shack-Hartmann wavefront analyser comprises a Fourier filter, a microlens array 41 and a matrix-type image sensor 42. The image sensor 42 is located in the image focal plane of the microlens array 41, both downstream of the Fourier filter. For example, the Fourier filter may comprise two lenses 43 and 45, combined with a pinhole mask 44. The image focus point F'₄₃ of the lens 43 is superimposed on the object focus point F₄₅ of the lens 45 so as to form an afocal optical combination. The pinhole mask 44 is then located at the focus points F'₄₃ and F₄₅. The aberrometer may be combined with an additional ametropia-compensating device (not represented) acting on the second part B2 of the return beam BR, enabling to optimize the aberration dynamic range of the aberrometer even in the presence of a strong ametropia in the eye 101 of the patient.

It is repeated that the disclosed system allows reliable convergence in determining the personalized values P of the parameters of the eye model thanks to the off-axis measurement configuration which is shared by the aberrometer and the optical coherence tomography device. Such configuration provides enough measurement information, in particular not-redundant information about the patient's eye, thanks to implementing at least one or multiple off-axis measurement configurations. System structure that combines aberrometry and optical coherence tomography allows simultaneous measurements, thereby participating in the reliability of the determined parameter values.

Although several embodiment variants have been mentioned in the above detailed description, still other variants and system adaptations may be implemented while maintaining the cited advantages. Finally, the disclosed system is compatible with any eye model, whatever their type and refinement level.

## Claims

1. A system (100) for determining a characterization of at least one parameter of an eye model for a patient's eye (101), the system comprising:
- an optical coherence tomography device,
- an aberrometer, and
- a double-pass channel (10), which is common to the optical coherence tomography device and the aberrometer,
wherein the double-pass channel (10) is arranged for injecting an illumination beam (B0) into the patient's eye (101) and recovering a return beam (BR) from the patient's eye both parallel to a common optical direction, during a system use,
the optical coherence tomography device is configured for providing measured values of an optical path length,
the aberrometer is configured for providing measured features of a wavefront,
and the system (100) is configured for determining the characterization of the at least one parameter from the measured values of the optical path length and the measured features of the wavefront relating to at least two angular positions of the common optical direction.

2. The system (100) of claim 1, further comprising:
- an ametropia-compensating device (30) arranged on an optical path segment that is common to a part of the illumination beam (B0) and to a part of the return beam (BR) which propagates to an interferometer and detector arrangement of the optical coherence tomography device, and such that the illumination beam is focused onto a retina (R) of the patient's eye (101) during the system use.

3. The system (100) of claim 2, further comprising a beam divider (60) arranged for directing a further part (B1) of the return beam (BR) to the interferometer and detector arrangement of the optical coherence tomography device, and directing another further part (B2) of the return beam to a wavefront analyser (40) of the aberrometer.

4. The system (100) of one of the preceding claims, wherein the double-pass channel (10) comprises:
- a scanning device at an entrance pupil (EP) of the double-pass channel (10), suitable for varying between the at least two angular positions of the common optical direction; and
- an afocal optical device (14) located between the entrance pupil (EP) and a sample pupil (SP) of the double-pass channel (10) dedicated to being superimposed on a pupil (OP) of the patient's eye (101) during the system use, so as to transmit both the illumination beam (B0) and the return beam (BR) between the entrance pupil and the sample pupil.

5. The system (100) of one of the preceding claims, further configured for controlling a variation of the common optical direction according to a continuous motion that comprises successive superpositions of the common optical direction with a central optical axis (A0) of the double-pass channel (10), and wherein the measured values of the optical path length and the measured features of the wavefront are measured at least one time between two successive superpositions of the common optical direction with the central optical axis.

6. The system (100) of one of claims 1 to 5, wherein the system is configured for:
repeatedly implementing the eye model according to tested characterizations of the at least one parameter, for calculating test results that comprise calculated values of the optical path length and calculated features of the wavefront for the at least two angular positions of the common optical direction; and
determining the characterization of the at least one parameter of the eye model from a best-match search carried out between:
- a target that comprises the measured values of the optical path length and the measured features of the wavefront for the at least two angular positions of the common optical direction, and
- the test results.

7. The system (100) of claim 6, wherein the system is further configured for carrying out the best-match search by computing values of a cost function that quantifies a discrepancy between the target and the test results.

8. The system (100) of one of claims 1 to 5, wherein the system is configured for implementing a relationship providing the characterization of the at least one parameter of the eye model from the measured values of the optical path length and the measured features of the wavefront relating to the at least two angular positions of the common optical direction.

9. The system (100) of one of the preceding claims, wherein the system is configured for determining the characterization of the at least one parameter, from statistical data that relate to characterizations possible for the at least one parameter of the eye model, clinical data of the patient and/or additional measurement data collected for the patient, in addition to the measured values of the optical path length and the measured features of the wavefront relating to the at least two angular positions of the common optical direction.

10. The system (100) of claim 9, wherein the statistical data include probability values and/or covariance values relating to the at least one parameter of the eye model.

11. The system (100) of one of the preceding claims, wherein the system is configured for determining the characterization of the at least one parameter separately for several eye models from one and the same measurement sequence executed for the patient's eye (101), and outputting respective values of a residual discrepancy for the eye models, each of the values of the residual discrepancy quantifying for the corresponding eye model a discrepancy between:
- modelled values of the optical path length and modelled features of the wavefront for the at least two angular positions of the common optical direction as resulting from inputting into the eye model the characterization of the at least one parameter obtained for this eye model, and
- the measured values of the optical path length and the measured features of the wavefront for the at least two angular positions of the common optical direction,
and the system (100) is further configured for selecting one of the eye models that has the value of the residual discrepancy corresponding to a least discrepancy.

12. A method for determining a characterization of at least one parameter of an eye model for a patient's eye (101), the method comprising:
/i/ providing a system (100) that comprises:
- an optical coherence tomography device,
- an aberrometer, and
- a double-pass channel (10), which is common to the optical coherence tomography device and the aberrometer,
wherein the double-pass channel (10) is arranged for injecting an illumination beam (B0) into the patient's eye (101) and recovering a return beam (BR) from the patient's eye both parallel to a common variable optical direction, during a system use,
the optical coherence tomography device is configured for providing measured values of an optical path length,
the aberrometer is configured for providing measured features of a wavefront,
and the system (100) is configured for determining the characterization of the at least one parameter, from measured the values of the optical path length and the measured features of the wavefront relating to at least two angular positions of the common optical direction;
/ii/ positioning the patient's eye (101) with respect to the system (100) and collecting the respective measured values of the optical path length and the respective measured features of the wavefront for the at least two angular positions of the common optical direction; and
/iii/ from input data that comprise the measured values of the optical path length and the measured features of the wavefront for the at least two angular positions of the common optical direction, determining the characterization of the at least one parameter of the eye model that relate to the patient's eye (101).

13. The method of claim 12, wherein a number of the at least two angular positions of the common optical direction is comprised between ten and one hundred.

14. The method of claim 12 or 13, wherein the system (100) is according to claim 8, and the relationship was obtained by a neural network previously trained with data of at least one of the following types: simulated data and experimental data.

15. The method of one of claims 12 to 14, wherein the at least one parameter describes an accommodation state of the patient's eye (101), a shape of a retina of the patient's eye, or a refractive index gradient of a crystalline lens of the patient's eye.
